# EUROPEAN PATENT APPLICATION

(11) **EP 4 234 542 A1**
(43) Date of publication of application: **30.08.2023**
(21) Application number: 22461507.0
(22) Date of filing: 28.02.2022
(51) Int. Cl.: C07D 285/28

(54) **A PROCESS FOR MANUFACTURING HIGH PURITY HYDROCHLOROTHIAZIDE**

(71) Applicant: Zaklady Farmaceutyczne Polpharma S.A., 83-200 Starogard Gdanski (PL)
(72) Inventor: PUNDA, Pawel, 83-200 Okole (PL); HALUSZCZUK, Adam Andrzej, 80-058 Gdansk (PL); JASINSKA, Agnieszka Paulina, 80-169 Gdansk (PL); KUROWSKI, Mariusz, 83-200 Starogard Gdanski (PL)

(57) **Abstract**

The present invention relates to a process for manufacturing high purity hydrochlorothiazide. In particular the present invention relates to a process for reducing amount of 6-chloro-4-nitroso-3,4-dihydro-2H-1,2,4-benzothiadiazide-7-sulfonamide-1,1-dioxide in a particulate hydrochlorothiazide and/or improving stability of a particulate hydrochlorothiazide against formation of 6-chloro-4-nitroso-3,4-dihydro-2H-1,2,4-benzothiadiazide-7-sulfonamide-1,1-dioxide, wherein a particulate hydrochlorothiazide is subjected to a treatment with a basic agent.

## Description

The present invention relates to a process for manufacturing high purity hydrochlorothiazide. In particular the present invention relates to a process for reducing amount of 6-chloro-4-nitroso-3,4-dihydro-2H-1,2,4-benzothiadiazide-7-sulfonamide-1,1-dioxide in a particulate hydrochlorothiazide and/or improving stability of a particulate hydrochlorothiazide against formation of 6-chloro-4-nitroso-3,4-dihydro-2H-1,2,4-benzothiadiazide-7-sulfonamide-1,1-dioxide.

### Background art

Hydrochlorothiazide, i.e. 6-chloro-3,4-dihydro-2H-1,2,4-benzothiadiazine-7-sulfonamide 1,1-dioxide, has the following structural formula (1):

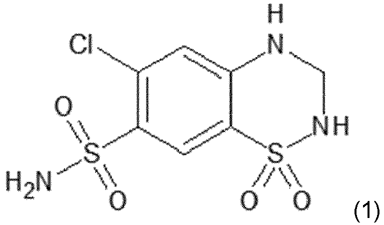

Hydrochlorothiazide is a diuretic drug that belongs to the thiazide class of diuretics and acts by inhibiting the kidney's ability to retain water. It is indicated alone or in combination for the management of hypertension, edema associated with congestive heart failure, hepatic cirrhosis, nephrotic syndrome, acute glomerulonephritis, chronic renal failure, and corticosteroid and estrogen therapy.

B. Gold and S. Mirvish, Toxicology and applied pharmacology 40, 131-136 (1977) describe that hydrochlorotiazide undergoes diazotization when treated with sodium nitrite under acidic conditions. It is disclosed that hydrochlorothiazide has three potential sites for nitrosation but only the 4-nitroso-derivative of hydrochlorothiazide i.e 6-chloro-4-nitroso-3,4-dihydro-2H-1,2,4-benzothiadiazide-7-sulfonamide-1,1-dioxide of formula (2) was formed in experiments.

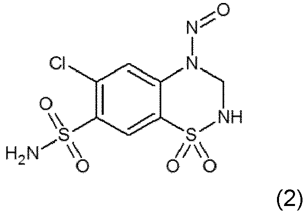

While in the discussion it is mentioned that the carcinogenicity of nitroso-derivative of hydrochlorothiazide is unknown, there is a general presumption that N-nitroso-compounds are at least potential carcinogens.

Yurii A. Simonov et al. , Nitrosation of hydrochlorothiazide and the modes of binding of the N-nitroso derivative with two macrocycles possessing an 18-membered crown ether cavity, Tetrahedron, vol. 26, Iss. 27, 6596-6601 (2005) also discuss the formation of 4-nitroso-derivative of hydrochlorothiazde in two possible ways, namely, by endogenous nitrogen oxide (NO) in the conditions of the oxidation reaction and by exogenous nitrites in the acidic medium. Similarly to Gold and Mirvish, it is concluded that 4-nitroso-derivative of hydrochlorothiazde has a carcinogenic potential.

US3163645 discloses a process for the preparation of hydrochlorothiazide by reacting 5-chloro-2,4-disulfamyl-aniline with paraformaldehyde in the presence of a mineral acid and a solvent. The mineral acid is selected from hydrochloric acid, hydrobromic acid and sulfuric acid. Hydrochlorothiazide obtained is recrystallized from water.

US3227710 discloses a process for the preparation of hydrochlorothiazide by reacting 4,6-dichloro-benzene-1,3-disulfonic acid dichloride with 30% aq. formaldehyde in the presence of saturated alcoholic ammonia solution at 150°C, acidifying the reaction mixture with hydrochloric acid followed by charcoalization and crystallization of hydrochlorothiazide from an alcoholic solvent.

US3267095 discloses a process for the preparation of hydrochlorothiazide by reacting 5-chloro-aniline 2,4-disulfonyl chloride with paraformaldehyde in the presence of gaseous hydrogen chloride in diethyl ether to give an intermediate which is further reacted with an anhydrous ammonia to give hydrochlorothiazide. Subsequent recrystallization from aqueous methanol gives pure hydrochlorothiazide.

US3043840 discloses a process for purification of hydrochlorothiazide by dissolving crude hydrochlorothiazide in an aqueous solution of ammonia or water-soluble primary or secondary amine, followed by neutralization using hydrochloric acid or washing with water to obtain pure hydrochlorothiazide.

US3164588 discloses a process for the preparation of hydrochlorothiazide by treating 6-chloro-7-sulfamyl-1,2,4-benzothiadiazine 1,1-dioxide with sodium hydroxide and formaldehyde followed by pH adjustment with hydrochloric acid and treatment with water and 35% ammonia solution to crystallize hydrochlorothiazide. In Example 2 there is described a washing of crystalline hydrochlorothiazide with diluted ammonia and with distilled water. The procedure is supposed to obtain hydrochlorothiazide with 53.5% yield and 1.0% diazotisables.

US3340150 discloses a process for the preparation of hydrochlorothiazide by reacting 5-chloro-2,4-disulfamyl-aniline with paraformaldehyde in the presence of HCl in diethylene glycol diethyl ether to give hydrochlorothiazide. Crude product is further recrystallized from water.

CN1421441 discloses a purification of hydrochlorothiazide using ammonia and alcoholic solvent system.

WO2007/026376 A1 discloses a process for the preparation of pure hydrochlorothiazide by reacting 4-amino-6-chloro-1,3-benzenedisulfonamide and paraformaldehyde in alcoholic solvent and adding a solution of inorganic acid in alcohol. The inorganic acid is selected from hydrochloric acid and sulfuric acid.

WO2007/026376 discloses the process which enables removal of two main known impurities of the prior art processes i.e. 4-amino-6-chloro-1,3-benzenesulfonamide and chlorothiazide.

WO2009/150497 A1 discloses a process for the preparation of hydrochlorothiazide which enables to reduce the content of dimer and trimer impurities in hydrochlorothiazide. The process involves reacting 5-chloro-2,4-disulfamyl aniline with paraformaldehyde in the absence of acid or base followed by purification by dissolving in aqueous solution of ammonia and sodium hydroxide and adjusting pH using hydrochloric acid.

As it has been mentioned above, due to a health risk related to their carcinogenicity, nitroso-compounds are of a very serious concern and their content in pharmaceutical products should be kept at possibly lowest level.

According to recommendations of CHMP of the EMA as contained in the assessment report "Nitrosamine impurities in human medicinal products" EMEA/H/A-5(3)/1490, Marketing Authorization Holders "should implement a control strategy regarding N-nitrosamines for their active substances and finished products, which should include current and prospective measures to minimise the risk of generation/contamination with any nitrosamine, including carry out risk evaluation/risk assessment of manufacturing processes of API (route of synthesis, starting materials, intermediates, raw materials) in view of potential formation of or contamination with N-nitrosamines, taking into account potential and confirmed root causes for the presence of N-nitrosamines in APIs." In case of "N-nitrosamines with insufficient substance specific data to derive a substance specific limit for lifetime exposure as recommended in ICH M7(R1), a class specific TTC (Treshold Toxicological Concern) for nitrosamines of 18 ng/d can be used as default option. This TTC has been derived from the Lhasa carcinogenic potency database and is considered a conservative and acceptable approach."

Since a maximum daily dose (MDD) for hydrochlorothiazide is 50 mg/day, when the above indicated TTC is taken into consideration, a maximum content of N-nitrosamines in that active ingredient should not exceed 0,36 ppm.

This puts a significant burden upon API manufacturers who should find methods of avoiding or eliminating N-nitroso-impurities in their products and if possible, to minimise a risk that during storage or formulation of pharmaceutical composition the content of N-nitroso-impurity would increase to an unacceptable level.

The inventors of the present invention have found that there are few factors which affect the formation of 4-nitroso derivative of hydrochlorothiazide.

It has been observed that not only chemical factors like presence of reactive forms of oxygen or nitrogen, but also mechanical stress causes formation of 4-nitroso derivative of hydrochlorothiazide to an unacceptable level. Avoiding of or reducing formation of 4-nitroso derivative of hydrochlorothiazide is very difficult since mechanical stress generally occurs during all conventional operations performed on hydrochlorothiazide. In particular, it was observed that simple mixing of hydrochlorothiazide, grinding, drying in mixer-dryer and micronization cause an increase in content of 4-nitroso derivative of hydrochlorothiazide.

Without wishing to be bound by any theory, it is hypothesized that mechanical stress causes local increase of temperature which in turn promotes formation of 4-nitroso derivative of hydrochlorothiazide on the surface of its particles. Recrystallization does not solve the problem since subsequent physical operations like mixing or micronization would be also necessary and would again promote formation of 4-nitroso derivative of hydrochlorothiazide.

The above problem has been solved by a process of the present invention.

### Description of the invention

As used herein, the terms "a", "an" and the are used herein to refer to one or more than one, i.e. to at least one.

As used herein, a particulate hydrochlorothiazide means hydrochlorothiazide in solid state having a form of solid particles, in particular a powder or an agglomerated powder, more preferably a powder, and even more preferably a crystalline powder. Depending on a process of providing the particulate hydrochlorothiazide to be subjected to the process of the present invention, the particulate hydrochlorothiazide can be dry or wet, i.e. contain some amount of solvent, for example water. It will be clear for a skilled person that, for example, the particulate hydrochlorothiazide for micronization would be provided as a dry substance while drying would be performed on wet particulate hydrochlorothiazide.

The terms "4-nitroso derivative of hydrochlorothiazide", "4-nitroso impurity", "N-nitroso hydrochlorothiazide" or "4-nitroso derivative" as used therein refer to 6-chloro-4-nitroso-3,4-dihydro-2H-1,2,4-benzothiadiazide-7-sulfonamide-1,1-dioxide and are used interchangeably.

As used herein, the term "reducing amount of 6-chloro-4-nitroso-3,4-dihydro-2H-1,2,4-benzothiadiazide-7-sulfonamide-1,1-dioxide in particulate hydrochlorothiazide" means decreasing a content of 6-chloro-4-nitroso-3,4-dihydro-2H-1,2,4-benzothiadiazide-7-sulfonamide-1,1-dioxide as compared with the particulate hydrochlorothiazide without a treatment with a basic agent according to the present invention. Amount of 4-nitroso impurity in hydrochlorothiazide can be determined using HPLC or UPLC.

As used herein, the term "improving stability of a particulate hydrochlorothiazide against formation of 6-chloro-4-nitroso-3,4-dihydro-2H-1,2,4-benzothiadiazide-7-sulfonamide-1,1-dioxide" means inhibiting/ reducing formation or making a formation of that impurity slower when subject the particulate hydrochlorothiazide to storing or to physical operations, e.g. mixing, manufacturing of formulation, as compared with said formation of 6-chloro-4-nitroso-3,4-dihydro-2H-1,2,4-benzothiadiazide-7-sulfonamide-1,1-dioxide during storing of or physical operations on particulate hydrochlorothiazide which was not subject to the process according to the present invention.

As used herein, the term "treatment of a particulate hydrochlorothiazide with a basic agent" means putting the particulate hydrochlorothiazide into a direct contact with the basic agent. During the treatment basically no changes occur in particulate form of hydrochlorothiazide, i.e. no significant change of solid state of matter of hydrochlorothiazide occurs.

As used herein, the term "a basic agent" means a compound or a mixture of compounds which after being dissolved in water is/are able to impart pH > 7 to the resulting solution, more preferably pH within a range of 10,0-13,5.

In the first aspect, the present invention relates to a process for reducing amount of 6-chloro-4-nitroso-3,4-dihydro-2H-1,2,4-benzothiadiazide-7-sulfonamide-1,1-dioxide (Formula (2)) in a particulate hydrochlorothiazide (Formula (1)) and/or improving stability of a particulate hydrochlorothiazide against formation of 6-chloro-4-nitroso-3,4-dihydro-2H-1,2,4-benzothiadiazide-7-sulfonamide-1,1-dioxide.

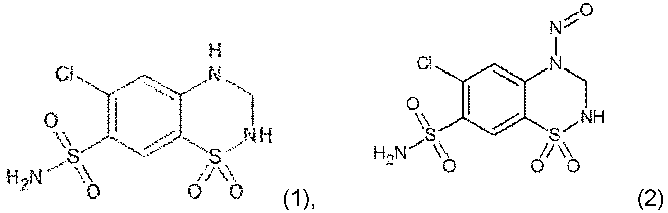

Accordingly the present invention provides a process for reducing amount of 6-chloro-4-nitroso-3,4-dihydro-2H-1,2,4-benzothiadiazide-7-sulfonamide-1,1-dioxide in a particulate hydrochlorothiazide and/or improving stability of a particulate hydrochlorothiazide against formation of 6-chloro-4-nitroso-3,4-dihydro-2H-1,2,4-benzothiadiazide-7-sulfonamide-1,1-dioxide, comprising the following steps:
(a) providing the particulate hydrochlorothiazide,
(b) a treatment of the particulate hydrochlorothiazide with a basic agent,
(c) optionally, one or more additional treatments of a product of step (b) with one or more basic agents, respectively,
wherein during the treatment of hydrochlorothiazide with the basic agent in step (b) and with one or more basic agents in optional step (c) hydrochlorothiazide remains in the particulate form, and wherein the treatment of hydrochlorothiazide with the basic agent in step (b) and with one or more basic agents in optional step (c) does not consist in washing the particulate hydrochlorothiazide with diluted ammonia.

The present inventors have surprisingly found that contacting the particulate hydrochlorothiazide with one or more basic agents during various physical operations allows to reduce amount of 4-nitroso impurity therein or prevent formation of that impurity allowing to keep its content in the final product within a safe and from regulatory point of view acceptable range. Unexpectedly, the treatment with one or more basic agents according to the present invention does not negatively affects the overall purity of hydrochlorothiazide, i.e. contacting with basic agent(s) according to the present invention does not cause degradation of hydrochlorothiazide and formation of any additional impurity, as confirmed by HPLC analysis. This is surprising in view of the prior art, where conditions of isolation the final hydrochlorothiazide are usually neutral.

The present inventors observed an interesting exception, wherein if the particulate hydrochlorothiazide is washed with aqueous ammonia, e.g. after isolation of solid hydrochlorothiazide as described in US3164588, Example 2, then the effect with respect to 4-nitroso impurity and generally, certain purity problems, is not satisfactory.

Moreover, the particulate hydrochlorothiazide obtained after such washing becomes sticky causing formation of undesired very coarse and non-homogenous material.

Since step (c) of the process according to the present invention is optional, it can be absent. In such a case the particulate hydrochlorothiazide as provided in step (a) is subject to the treatment with the basic agent in step (b) and no further treatment(s) using basic agent(s) are performed.

If step (c) is present in the process according to the invention, the one or more basic agents used for one or more treatments in step (c) can be the same or different as the basic agent in step (b) and can have the same or different physical form selected from a gaseous and liquid forms. Analogously, each of one or more basic agents used for one or more treatments in step (c) can be the same or different and can have the same or different physical form selected from the gaseous and liquid forms and which have been presented further in more detail. Furthermore, the treatment of step (b) and one or more treatments of step (c) can be performed using the same or different procedure as defined further.

The basic agent in step (b) of the process according to the present invention can have a gaseous or a liquid form.

In one embodiment of the process of the present invention step (c) is present and, as mentioned above, the basic agent of step (b) and one or more basic agents of step (c), independently of each other, are the same or different and have the same or different form selected from the gaseous and liquid form.

In another embodiment of the process according to the present invention, the basic agent of step (b) and/or the one or more basic agents of step (c), if present, have the gaseous form. Preferably, the basic agent in gaseous form is ammonia.

Preferably, when the basic agent has the gaseous form it is used as a gaseous mixture with an inert gas, wherein concentration of said basic agent in the gaseous mixture with inert gas is within a range of 0,05 - 10% v/v, preferably 0,1 - 8% v/v, more preferably 0,2 - 3% v/v, in relation to a total volume of the gaseous mixture. An inert gas for mixtures with the gaseous basic agent, in particular with ammonia, is selected from a group consisting of nitrogen, argon and carbon dioxide, preferably an inert gas is nitrogen. The concentration ranges as indicated above generally for the inert gas, clearly apply also to nitrogen.

In the most preferred embodiment of using the basic agent in gaseous form in the process according to the present invention, the basic agent in gaseous form as used in step (b) and/or in step (c), if present, is a gaseous mixture of ammonia with gaseous nitrogen, further preferably wherein concentration of ammonia in the gaseous mixture with nitrogen is within a range of 0,05 - 10% v/v, preferably 0,1 - 8% v/v, more preferably 0,2 - 3% v/v, in relation to a total volume of the gaseous mixture.

According to the present invention, the treatment of the particulate hydrochlorothiazide with the basic agent in gaseous form in step (b) and/or in step (c), if present, consists in:
- flushing the particulate hydrochlorothiazide with the basic agent in gaseous form,
- storing the particulate hydrochlorothiazide in the presence of the basic agent in gaseous form,
- drying the particulate hydrochlorothiazide in the presence of the basic agent in gaseous form,
- mixing the particulate hydrochlorothiazide in the presence of the basic agent in gaseous form, or
- micronizing the particulate hydrochlorothiazide in the presence the basic agent in gaseous form, preferably as a milling gas.

Flushing the particulate hydrochlorothiazide with the basic agent in gaseous form, as mentioned above, can be performed, e.g. by passing said basic agent in gaseous form through a bed/layer of the particulate hydrochlorothiazide.

Storing the particulate hydrochlorothiazide in the presence of the basic agent in gaseous form, as mentioned above, can be carried out in a basically tightly closed bag and/or container, for example in a polyethylene bag or drum, wherein said basic agent in gaseous form forms an atmosphere above the particulate hydrochlorothiazide.

Drying the particulate hydrochlorothiazide in the presence of the basic agent in gaseous form, as mentioned above, can be performed using conventional equipment, e.g. drying oven, mixer-dryer, fluidized bed dryer, wherein said basic agent in gaseous form forms an atmosphere surrounding the particulate hydrochlorothiazide.

Mixing the particulate hydrochlorothiazide in the presence of the basic agent in gaseous form, as mentioned above, can be performed using conventional equipment, e.g. a double cone solid blender, wherein said basic agent in gaseous form forms is loaded together with the particulate hydrochlorothiazide and forms an atmosphere surrounding the particulate hydrochlorothiazide.

Micronizing the particulate hydrochlorothiazide in the presence of the basic agent as a milling gas, as mentioned above, can be performed using conventional equipment, e.g. jet mill, wherein said basic agent in gaseous, pressurized form is used to move the particles at a high speed forcing them to collide with each other and break into small uniform-size particle.

Preferably, for the above mentioned operations, i.e. flushing, storing, drying, mixing and micronizing, the basic agent in gaseous form is a mixture of ammonia with nitrogen, wherein the concentration of ammonia in said mixture is within a range of 0,05-10% v/v, preferably 0,1-8% v/v, more preferably 0,2 - 3% v/v, in relation to the total volume of gaseous mixture.

In another embodiment, wherein the basic agent in gaseous form used in the process of the present invention is ammonia, the treatment of the particulate hydrochlorothiazide with ammonia in step b) and/or step c), if present, consists in contacting the particulate hydrochlorothiazide with a solid ammonium salt evolving ammonia during decomposition. Preferably, the solid ammonium salt evolving ammonia during decomposition is selected from a group consisting of ammonium acetate, ammonium carbonate, ammonium hydrogen carbonate, and mixtures thereof, more preferably said salt is ammonium acetate.

In the preferred variant of the above embodiment, the amount of the solid ammonium salt is within a range of 1 - 50% w/w, preferably within a range of 2-5% w/w, in relation to the weight of the particulate hydrochlorothiazide subject to such treatment.

In particular, the above embodiment of contacting the particulate hydrochlorothiazide with a solid ammonium salt evolving ammonia during decomposition, preferably with ammonium acetate, is performed as
- mixing the particulate hydrochlorothiazide with the solid ammonium salt followed by heating the resulting solid mixture to decompose the ammonium salt while evolving gaseous ammonia, or
- co-micronizing the particulate hydrochlorothiazide with the solid ammonium salt followed by heating the resulting co-micronizate to decompose the ammonium salt while evolving gaseous ammonia.

For both above mentioned operations, heating the resulting solid mixture or the resulting co-micronizate is carried out at a temperature within a range of 50-120°C under vacuum.

In another embodiment of the process according to the present invention, the basic agent of step (b) and/or the one or more basic agents of step (c), if present, have the liquid form.

Preferably, when the basic agent of step (b) and/or the one or more basic agents of step (c), if present, have the liquid form, it is selected from an aqueous solution of one or more alkali metal hydroxides, one or more alkaline earth metal hydroxides, one or more alkali metal carbonates and/or one or more alkali metal hydrogen carbonates.

Preferably, when the basic agent of step (b) and/or the one or more basic agents of step (c), if present, have the liquid form, it is selected from an aqueous solution of one or more alkali metal hydroxides and/or one or more alkaline earth metal hydroxides.

Preferably, the alkali metal hydroxide in the above embodiment is selected from a group consisting of lithium, potassium and sodium hydroxide and alkaline earth metal hydroxide is calcium hydroxide. More preferably, the basic agent of step (b) and/or the one or more basic agents of step (c), if present, is an aqueous solution of sodium hydroxide.

Preferably, a concentration of one or more alkali metal hydroxides and/or one or more alkaline earth metal hydroxides in aqueous solution of the above embodiment is within a range of 0,001-10% w/w, preferably 0,01-5% w/w, and more preferably 0,02-0,5% w/w, in relation to the total weight of the aqueous solution. Volume of the aqueous solution as mentioned above should ensure an efficient contact of the particulate hydrochlorothiazide with the aqueous solution. For example, when washing of the particulate hydrochlorothiazide during filtration is performed, then the aqueous solution should cover a filter cake.

According to the present invention, the treatment of the particulate hydrochlorothiazide with the basic agent in liquid form in step (b) and/or in step (c), if present, consists in:
- washing the particulate hydrochlorothiazide with the basic agent in liquid form during filtration, or
- suspending the particulate hydrochlorothiazide in that basic agent in liquid form followed by filtration.

The above operations can be carried out using conventional equipment. For example, washing can be performed during filtration in a static filter or in a centrifuge.

Washing of the particulate hydrochlorothiazide with the basic agent in liquid form, e.g. with an aqueous solution of sodium hydroxide, during filtration, may be carried out using one portion of the liquid or the liquid can be divided into portions. Dividing the liquid into portions for stepwise washing the particulate hydrochlorothiazide should be regarded one treatment.

In the preferred embodiment, the process for reducing amount of 6-chloro-4-nitroso-3,4-dihydro-2H-1,2,4-benzothiadiazide-7-sulfonamide-1,1-dioxide in a particulate hydrochlorothiazide and/or improving stability of a particulate hydrochlorothiazide against formation of 6-chloro-4-nitroso-3,4-dihydro-2H-1,2,4-benzothiadiazide-7-sulfonamide-1,1-dioxide, comprising the following steps:
(a) providing the particulate hydrochlorothiazide,
(b) a treatment of the particulate hydrochlorothiazide with an aqueous solution of sodium hydroxide of concentration within a range of 0,001-10% w/w, preferably 0,01-5% w/w, and more preferably 0,02-0,5% w/w, in relation to the total weight of the aqueous solution, and wherein the treatment of the particulate hydrochlorothiazide consists in washing the particulate hydrochlorothiazide with said aqueous solution of sodium hydroxide during filtration.

In the above embodiment, apart from the main purpose relating to 4-nitroso derivative of hydrochlorothiazide, hydrochlorothiazide is obtained with a very low content of chlorides which can remain after synthesis of hydrochlorothiazide.

In another preferred embodiment, the process for reducing amount of 6-chloro-4-nitroso-3,4-dihydro-2H-1,2,4-benzothiadiazide-7-sulfonamide-1,1-dioxide in a particulate hydrochlorothiazide and/or improving stability of a particulate hydrochlorothiazide against formation of 6-chloro-4-nitroso-3,4-dihydro-2H-1,2,4-benzothiadiazide-7-sulfonamide-1,1-dioxide, comprising the following steps:
(a) providing the particulate hydrochlorothiazide,
(b) a treatment of the particulate hydrochlorothiazide with a gaseous mixture of ammonia and nitrogen, wherein a concentration of ammonia in said gaseous mixture is within a range of 0,05-10% v/v, preferably 0,1 -8% v/v, more preferably 0,2 - 3% v/v, in relation to a total volume of said gaseous mixture, and wherein the treatment of the particulate hydrochlorothiazide consists in micronizing the particulate hydrochlorothiazide in the presence of said mixture as a milling gas.

In the second aspect, the present invention provides a process for reducing amount of 6-chloro-4-nitroso-3,4-dihydro-2H-1,2,4-benzothiadiazide-7-sulfonamide-1,1-dioxide in a particulate hydrochlorothiazide consisting in:
a1) providing the particulate hydrochlorothiazide containing 6-chloro-4-nitroso-3,4-dihydro-2H-1,2,4-benzothiadiazide-7-sulfonamide-1,1-dioxide, and
b2) heating the particulate hydrochlorothiazide to 120-150°C, preferably to 140°C.

The process as defined above allows for decreasing significantly an amount of 4-nitroso hydrochlorothiazide in the particulate hydrochlorothiazide containing that impurity. Surprisingly, applying the above conditions allows for selective decomposition of 4-nitroso impurity in the particulate hydrochlorothiazide without deterioration in overall purity and quality of the particulate hydrochlorothiazide.

Preferably, the heating of step b2) is carried out for 10-20 hours.

The particulate hydrochlorothiazide for step a) of the process according to the first aspect of the present invention and/or for step a1) according to the second aspect of the present invention can be provided by any process known in the art.

The particulate hydrochlorothiazide for the above mentioned steps of any embodiments and variants of the processes according to the present invention may be also manufactured in a process, comprising the following steps:
(i) reacting 6-chloro-4-amino-1,3-benzenedisulfonamide with paraformaldehyde in the presence of water, a mineral acid and optionally an antioxidant in reflux to obtain a solution containing hydrochlorothiazide,
(ii) cooling the solution obtained in step (i) to a room temperature,
(iii) filtering off the particulate hydrochlorothiazide,
(iv) optionally, drying the particulate hydrochlorothiazide.

Preferably, a mineral acid used in step (i) is hydrochloric acid or sulfuric acid, most preferably sulfuric acid.

The antioxidant, if used in step (i), is preferably selected from ascorbic acid, citric acid, lipoic acid, BHT and/or tocopherol. It is particularly preferred when the antioxidant is ascorbic acid.

Suitable temperature for carrying out the reaction of step (i) ranges from 90 to 100°C. Preferably, step (i) is carried out at a temperature of from 98 to 100°C.

Drying the particulate hydrochlorothiazide obtained in step (iv), if present, is carried out at a temperature of from 80 to 120 °C. Preferably, drying is carried out at a temperature of from 90 to 100°C.

Alternatively, the particulate hydrochlorothiazide for step a) of the process according to the first aspect of the present invention and/or for step a1) according to the second aspect of the present in any of embodiments and variants of the processes according to the present invention, can be manufactured in a process, comprising the following steps:
(v) providing a solid hydrochlorothiazide,
(vi) dissolving the solid hydrochlorothiazide of step (v) in an aqueous solution of sodium hydroxide and ammonia,
(vii) optionally, adding an antioxidant to the solution of step (vi),
(viii) adding an aqueous solution of hydrochloric acid to the solution of step (vii) to adjust the pH value of said solution to 6 - 7,
(ix) filtering off the product precipitated in step (viii),
(x) optionally, drying the product of step (ix),
to obtain the particulate hydrochlorothiazide.

The antioxidant of step (vii), if present, is preferably selected from ascorbic acid, citric acid, lipoic acid, BHT and/or tocopherol. It is particularly preferred when the antioxidant is ascorbic acid.

Drying of the precipitated product (hydrochlorothiazide) in step (x), if present, is carried out at a temperature of from 80 to 120 °C. Preferably, drying is carried out at a temperature of from 90 to 100°C.

In the third aspect the present invention relates to a pharmaceutical composition containing a particulate hydrochlorothiazide, as obtained according to the process of the first aspect of the present invention or as obtained according to the process of the second aspect of the present invention, in combination with one or more pharmaceutically acceptable excipient(s).

In the preferred embodiment of the above third aspect, the pharmaceutical composition is formulated into a solid unit dosage form for oral administration, preferably into a tablet.

Pharmaceutically acceptable excipients which can be contemplated for use in the pharmaceutical composition of the present invention are conventional and known to the skilled person. For example, pharmaceutically acceptable excipients are described in P.J. Sheskey (Ed.), Handbook of Pharmaceutical Excipients: Edition 9, Pharmaceutical Press, 2020.

The present invention will now be described with reference to the following examples, which are not intended to be limiting.

### EXAMPLES

Conditions of UPLC analyses for detecting of 6-chloro-4-nitroso-3,4-dihydro-2H-1,2,4-benzothiadiazide-7-sulfonamide-1,1-dioxide in hydrochlorothiazide:

| Equipment | UPLC apparatus equipped with MS detector, adjusted to ultra-performance liquid chromatography | | |
|---|---|---|---|
| Column | Acquity UPLC HSS T3; 2.1 × 100 mm; particle size 1,8 µm (Waters, cat. No. 186003539) | | |
| Mobile phase A | 0,005% formic acid and 5 % THF in water | | |
| Mobile phase B | 0,005% formic acid in acetonitrile | | |

| | Time [min] | Mobile phase A [%] | Mobile phase B [%] |
|---|---|---|---|
| | 0.0 | 85 | 15 |
| Gradient | 8.0 | 70 | 30 |
| | 8.5 | 85 | 15 |
| | 10.0 | 85 | 15 |
| Acquisition time | 10.0 minutes | | |
| Mobile phase flow | 0.30 ml/min | | |
| Column temperature | 25°C | | |
| Autosampler temperature | 5°C | | |
| Injection volume | 2.0 µl | | |
| Diluent | acetonitrile : water (90:10; v/v) | | |
| Retention time (RT) of 6-chloro-4-nitroso-3,4-dihydro-2H-1,2,4-benzothiadiazide-7-sulfonamide-1,1-dioxide | 6 min | | |

### Example 1 - Preparation of the particulate hydrochlorothiazide

To a mixture of 35,8 g of sulfuric acid in 20 I of water, 500,6 g of 4-amino-6-chlorobenzene-1,3-disulphonamide acetamide was added followed by adding 59,2 g of paraformaldehyde. The mixture was heated and refluxed for 2 hours and then cooled to room temperature within 3 hours. Product was filtered off, washed with 6000 ml of water and dried under vacuum at 100 °C to obtain the particulate hydrochlorothiazide.
Yield: 500,0 g (88 %)
6-chloro-4-nitroso-3,4-dihydro-2H-1,2,4-benzothiadiazide-7-sulfonamide-1,1-dioxide by UPLC: 0,008 ppm

### Example 2 - Micronization of the particulate hydrochlorothiazide using gaseous nitrogen as milling gas

Part of hydrochlorothiazide of Example 1 was subjected to micronization using a jet mill. The process involves the use of pressurized gas to move particles at a high speed forcing them to collide with each other and break into small uniform -size particle.

Micronization parameters :
Gas pressure: 12 bar
Milling gas (N₂) feed: 40 Nm³/h
6-chloro-4-nitroso-3,4-dihydro-2H-1,2,4-benzothiadiazide-7-sulfonamide-1,1-dioxide by UPLC: 0,686 ppm

### Example 3 - Micronization of the particulate hydrochlorothiazide using a gaseous mixture of ammonia and nitrogen as a milling gas

Part of hydrochlorothiazide of Example 1 was subjected to micronization using a jet mill. The process involves the use of pressurized gas to move particles at a high speed forcing them to collide with each other and break into small uniform-size particle. Apart from milling gas, micronization parameters were the same as in Example 2.

Micronization parameters :
Gas pressure: 12 bar
Milling gas (N₂) feed: 40 Nm³/h
Ammonia gas feed : 100 dm³/h
6-chloro-4-nitroso-3,4-dihydro-2H-1,2,4-benzothiadiazide-7-sulfonamide-1,1-dioxide by UPLC: 0,013 ppm

### Example 4 - Preparation of the particulate hydrochlorothiazide

25 g of Hydrochlorothiazide of Example 1 were added to a solution of 8,18 g of NaOH in 175 ml of water at room temperature. Resulting mixture was heated to about 30°C and then 5 ml of 25% aqueous solution of ammonia were added. The reaction mixture was stirred for 1 hour, followed by adjusting pH value to about 6,5 using concentrated hydrochloric acid (37% w/w in water). To the reaction mass 125 ml of water added and the whole was stirred for additional hour at 40°C. The solid product was filtered off, washed with 480 ml of water and dried under vacuum at 100°C to obtain the particulate hydrochlorothiazide.
Yield: 22,8 g (91,2%)
6-chloro-4-nitroso-3,4-dihydro-2H-1,2,4-benzothiadiazide-7-sulfonamide-1,1-dioxide by UPLC: 0,013 ppm

### Example 5 - Washing the particulate hydrochlorothiazide with aqueous solution of sodium hydroxide

25 g of Hydrochlorothiazide of Example 1 were added to the solution of 8,18 g of NaOH in 175 ml of water at room temperature. Resulting mixture was heated to about 30°C and 5 ml of 25% aqueous solution of ammonia were added. The reaction mixture was then stirred for 1 hour and then pH value was adjusted to about 6,5 using concentrated hydrochloric acid. To the reaction mass 125 ml of water was added and the whole was stirred for additional hour at 40 °C. Product was filtered off, washed with 480 ml of water and 50 ml of 0,2% aqueous NaOH solution, then dried under vacuum at 100°C.
Yield: 22,9 g (91,6%)
6-chloro-4-nitroso-3,4-dihydro-2H-1,2,4-benzothiadiazide-7-sulfonamide-1,1-dioxide by UPLC: 0,005 ppm

### Example 6 - Washing the particulate hydrochlorothiazide with aqueous solution of sodium hydroxide

25 g of Hydrochlorothiazide of Example 1 were added to the solution of 8,18 g of NaOH in 175 ml of water at room temperature. Resulting mixture was heated to about 30°C and 5 ml of 25% aqueous solution of ammonia were added. The reaction mixture was then stirred for 1 hour and then pH value was adjusted to about 6,5 using concentrated hydrochloric acid. To the reaction mass 125 ml of water was added and the whole was stirred for additional hour at 40°C. Product was filtered off, washed with 480 ml of water and 50 ml of 0,5% aqueous NaOH solution, then dried under vacuum at 100°C.
Yield: 22,5 g (90,0%)
6-chloro-4-nitroso-3,4-dihydro-2H-1,2,4-benzothiadiazide-7-sulfonamide-1,1-dioxide by UPLC: 0,001

### Example 7 - Micronization of the particulate hydrochlorothiazide using gaseous nitrogen as milling gas

Hydrochlorothiazide obtained in Example 4 was subjected to micronization using a jet mill. The process involves the use of pressurized gas to move particles at a high speed forcing them to collide with each other and break into small uniform-size particle.

Micronization parameters:
Gas pressure: 12 bar
Milling gas (N₂) feed: 40 Nm³/h
Final content of 6-chloro-4-nitroso-3,4-dihydro-2H-1,2,4-benzothiadiazide-7-sulfonamide-1,1-dioxide by UPLC: 1,119 ppm

### Example 8 - Micronization of the particulate hydrochlorothiazide using a gaseous mixture of ammonia and nitrogen as a milling gas

Hydrochlorothiazide obtained in example 4 was subjected to micronization process using jet mill. The process involves the use of pressurized gas to move particles at a high speed forcing them to collide with each other and break into small uniform-size particle. Apart from milling gas, micronization parameters were the same as in Example 7.

Micronization parameters:
Gas pressure: 12 bar
Milling gas (N₂) feed: 40 Nm³/h
Ammonia gas feed : 100 dm³/h
Final content of 6-chloro-4-nitroso-3,4-dihydro-2H-1,2,4-benzothiadiazide-7-sulfonamide-1,1-dioxide by UPLC: 0,006 ppm

### Example 9 - Micronization of the particulate hydrochlorothiazide using gaseous nitrogen as milling gas

Hydrochlorothiazide obtained in Example 5 was subjected to micronization using a jet mill. The process involves the use of pressurized gas to move particles at a high speed forcing them to collide with each other and break into small uniform-size particle.

Micronization parameters:
Gas pressure: 12 bar
Milling gas (N₂) feed: 40 Nm³/h
6-chloro-4-nitroso-3,4-dihydro-2H-1,2,4-benzothiadiazide-7-sulfonamide-1,1-dioxide by UPLC: 0,150 ppm

### Example 10 - Micronization of the particulate hydrochlorothiazide using a gaseous mixture of ammonia and nitrogen as a milling gas

Hydrochlorothiazide obtained in Example 5 was subjected to micronization using a jet mill. The process involves the use of pressurized gas to move particles at a high speed forcing them to collide with each other and break into small uniform-size particle. Apart from milling gas, micronization parameters were the same as in Example 9.

Micronization parameters:
Gas pressure: 12 bar
Milling gas (N₂) feed: 40 Nm³/h
Ammonia gas feed : 100 dm³/h
6-chloro-4-nitroso-3,4-dihydro-2H-1,2,4-benzothiadiazide-7-sulfonamide-1,1-dioxide by UPLC: 0,009 ppm

### Example 11 - Micronization of the particulate hydrochlorothiazide using a gaseous mixture of ammonia and nitrogen as a milling gas

Hydrochlorothiazide obtained in Example 6 was subjected to micronization using a jet mill. The process involves the use of pressurized gas to move particles at a high speed forcing them to collide with each other and break into small uniform-size particle.

Micronization parameters:
Gas pressure: 12 bar
Milling gas (N₂) feed: 40 Nm³/h
Ammonia gas feed : 100 dm³/h
6-chloro-4-nitroso-3,4-dihydro-2H-1,2,4-benzothiadiazide-7-sulfonamide-1,1-dioxide by UPLC: 0,006 ppm

### Example 12 - Micronization of the particulate hydrochlorothiazide using gaseous nitrogen as milling gas and storing under ammonia atmosphere

Hydrochlorothiazide obtained in Example 1 was subjected to micronization using jet mill. The process involves the use of pressurized gas to move particles at a high speed forcing them to collide with each other and break into small uniform-size particle.

Micronisation parameters:
Gas pressure: 12 bar
Milling gas (N2) feed: 40 Nm³/h
6-chloro-4-nitroso-3,4-dihydro-2H-1,2,4-benzothiadiazide-7-sulfonamide-1,1-dioxide by UPLC: 0,686 ppm

The micronized hydrochlorothiazide was purged with a gaseous mixture of ammonia and nitrogen 0,2% v/v and stored in a PPE bag.

6-chloro-4-nitroso-3,4-dihydro-2H-1,2,4-benzothiadiazide-7-sulfonamide-1,1-dioxide by UPLC after 3 hours' storing: 0,168 ppm

### Example 13 - Co-micronization of particulate hydrochlorothiazide with ammonium acetate

25 g of Hydrochlorothiazide obtained in Example 1 was mixed with 2,5 g of ammonium acetate and subjected to micronization using jet mill. The process involves the use of pressurized gas to move particles at a high speed forcing them to collide with each other and break into small uniform-size particle.

Micronization parameters:
Gas pressure: 12 bar
Milling gas (N₂) feed: 40 Nm³/h
6-chloro-4-nitroso-3,4-dihydro-2H-1,2,4-benzothiadiazide-7-sulfonamide-1,1-dioxide by UPLC directly after micronization: 0,094 ppm

The micronized product was heated to 100° under vacuum to remove ammonium acetate.

6-chloro-4-nitroso-3,4-dihydro-2H-1,2,4-benzothiadiazide-7-sulfonamide-1,1-dioxide by UPLC after decomposition of ammonium acetate: 0,057 ppm

### Example 14 - Heating the particulate hydrochlorothiazide

Hydrochlorothiazide obtained in Example 1 was subjected to micronization using jet mill. The process involves the use of pressurized gas to move particles at a high speed forcing them to collide with each other and break into small uniform-size particle.

Micronization parameters:
Gas pressure: 12 bar
Milling gas (N₂) feed: 40 Nm³/h
6-chloro-4-nitroso-3,4-dihydro-2H-1,2,4-benzothiadiazide-7-sulfonamide-1,1-dioxide by UPLC after micronization: 0,686 ppm

The obtained product was collected and subjected to heating in a mixer-dryer under vacuum at 140°C.

6-chloro-4-nitroso-3,4-dihydro-2H-1,2,4-benzothiadiazide-7-sulfonamide-1,1-dioxide by UPLC after heating: 0,135 ppm

### Example 15 - Pharmaceutical composition of the particulate hydrochlorothiazide

Tablets were prepared as follows:
The particulate hydrochlorothiazide, lactose monohydrate, maize starch, talc and colloidal silica anhydrous were passed through a 1,7 mm sieve. The ingredients from the sieving step were mixed in a blender for 20 min at 25 RPM (rotations per minute). Magnesium stearate was then added and the powder mixture was mixed for 7 min at 12 RPM. The resulting blend was compressed using a laboratory rotary tablet press equipped with a round flat punches (8mm diameter) at a compression force of 10-157 kN. The composition of the tablets is shown in Table 1.

**Table 1.**

| Ingredient | Quantitity (mg/tablet) | wt % / tablet |
|---|---|---|
| Hydrochlorothiazide | 25 | 12,50 |
| Lactose monohydrate | 130,40 | 65,20 |
| maze starch | 40 | 20,00 |
| Talc | 2 | 1,00 |
| Colloidal silica anhydrous | 1 | 0,50 |
| Magnesium stearate | 1,60 | 0,80 |
| Total: | 300,00 | 100,00 |

Content of 6-chloro-4-nitroso-3,4-dihydro-2H-1,2,4-benzothiadiazide-7-sulfonamide-1,1 - dioxide was analysed directly after tableting. Results are presented in Table 2.

**Table 2.**

| Particulate hydrochlorothiazide | 6-chloro-4-nitroso-3,4-dihydro-2H-1,2,4-benzothiadiazide-7-sulfonamide-1,1-dioxide by UPLC in the particulate hydrochlorothiazide used for preparation of tablets [ppm] | 6-chloro-4-nitroso-3,4-dihydro-2H-1,2,4-benzothiadiazide-7-sulfonamide-1,1-dioxide by UPLC in the tablet [ppm] |
|---|---|---|
| Example 2 | 0,686 | 1,308 |
| (micronization of the particulate hydrochlorothiazide using N₂ as a milling gas) | | |
| Example 3 | 0,013 | 0,017 |
| (micronization of the particulate hydrochlorothiazide using a gaseous mixture of ammonia and nitrogen) | | |
| Example 5 | 0,005 | 0,012 |
| (washing the particulate hydrochlorothiazide with 0,2% aqueous solution of NaOH) | | |
| Example 6 | 0,001 | 0,010 |
| (washing the particulate hydrochlorothiazide with 0,5 % aqueous solution of NaOH) | | |
| Example 7 | 1,119 | 1,400 |
| (micronization of the particulate hydrochlorothiazide using N₂ as milling gas) | | |
| Example 8 | 0,006 | 0,017 |
| (micronization of the particulate hydrochlorothiazide using a gaseous mixture of ammonia and nitrogen as a milling gas) | | |
| Example 10 | 0,009 | 0,015 |
| (micronization of the particulate hydrochlorothiazide using a gaseous mixture of ammonia and nitrogen as a milling gas) | | |

Results show that in the tablets manufactured from the particulate hydrochlorothiazide which was subject to at least one treatment with a basic agent according to the present invention the content of 6-chloro-4-nitroso-3,4-dihydro-2H-1,2,4-benzothiadiazide-7-sulfonamide-1,1-dioxide is kept at a very low and safe level.

### Stability test

A test for chemical stability of tablets containing hydrochlorothiazide was performed as follows: Samples of tablets were exposed to a relative humidity of 75% (+/- 5%) at 40°C (+/- 2°C) for 4 weeks and the level of 6-chloro-4-nitroso-3,4-dihydro-2H-1,2,4-benzothiadiazide-7-sulfonamide-1,1-dioxide was checked at time 0 and after 4 weeks. Results are presented in Table 3.

**Table 3.**

| Particulate hydrochlorothiazide | 6-chloro-4-nitroso-3,4-dihydro-2H-1,2,4-benzothiadiazide-7-sulfonamide-1,1-dioxide by UPLC in the tablet at time 0 [ppm] | 6-chloro-4-nitroso-3,4-dihydro-2H-1,2,4-benzothiadiazide-7-sulfonamide-1,1-dioxide by UPLC in the tablet after 4 weeks [ppm] |
|---|---|---|
| Example 2 | 1,308 | 1,561 |
| (micronization of the particulate hydrochlorothiazide using N₂ as a milling gas) | | |
| Example 3 | 0,017 | 0,018 |
| (micronization of the particulate hydrochlorothiazide using a gaseous mixture of ammonia and nitrogen) | | |
| Example 5 | 0,011 | 0,014 |
| (washing the particulate hydrochlorothiazide with 0,2% aqueous solution of NaOH) | | |
| Example 6 | 0,010 | 0,011 |
| (washing the particulate hydrochlorothiazide with 0,5 % aqueous solution of NaOH) | | |
| Example 7 | 1,400 | 1,690 |
| (micronization of the particulate hydrochlorothiazide using N₂ as milling gas) | | |
| Example 8 | 0,017 | 0,015 |
| (micronization of the particulate hydrochlorothiazide using a gaseous mixture of ammonia and nitrogen as a milling gas) | | |
| Example 10 | 0,015 | 0,015 |
| (micronization of the particulate hydrochlorothiazide using a gaseous mixture of ammonia and nitrogen as a milling gas) | | |

The above results show that in the tablets manufactured from the particulate hydrochlorothiazide which was subject to at least one treatment with a basic agent according to the present invention the content of 6-chloro-4-nitroso-3,4-dihydro-2H-1,2,4-benzothiadiazide-7-sulfonamide-1,1-dioxide is kept at a very low and safe level after storing for 4 weeks at relative humidity of 75% (+/- 5%) at 40°C (+/- 2°C).

## Claims

1. A process for reducing amount of 6-chloro-4-nitroso-3,4-dihydro-2H-1,2,4-benzothiadiazide-7-sulfonamide-1,1-dioxide in a particulate hydrochlorothiazide and/or improving stability of a particulate hydrochlorothiazide against formation of 6-chloro-4-nitroso-3,4-dihydro-2H-1,2,4-benzothiadiazide-7-sulfonamide-1,1-dioxide, comprising the following steps:
(a) providing the particulate hydrochlorothiazide,
(b) a treatment of the particulate hydrochlorothiazide with a basic agent, and
(c) optionally, one or more additional treatments of a product of step (b) with one or more basic agents, respectively,
wherein during the treatment of hydrochlorothiazide with the basic agent in step (b) and with one or more basic agents in optional step (c) hydrochlorothiazide remains in the particulate form, and wherein the treatment of hydrochlorothiazide with the basic agent in step (b) and with one or more basic agents in optional step (c) does not consist in washing the particulate HCTZ with diluted ammonia.

2. The process according to claim 1, wherein the basic agent in step (b) has a gaseous or liquid form.

3. The process according to claims 1 or 2, wherein step (c) is present and wherein the basic agent of step (b) and one or more basic agents of step (c), independently of each other, are the same or different and have the same or different form selected from the gaseous and liquid form.

4. The process according to claims 2 or 3, wherein the basic agent of step (b) and/or the one or more basic agents of step (c) have the gaseous form.

5. The process according to any of claims 2 to 4, wherein the basic agent of step (b) and/or the one or more basic agents of step (c) is ammonia.

6. The process according to any of claims 2 to 5, wherein the basic agent in gaseous form is used as a gaseous mixture with an inert gas, wherein concentration of the basic agent in the gaseous mixture with inert gas is within a range of 0,05 - 10% v/v, preferably 0,1 - 8% v/v, more preferably 0,2 - 3% v/v, in relation to a total volume of the gaseous mixture.

7. The process according to any of claims 5 to 6, wherein an inert gas is selected from a group consisting of nitrogen, argon, carbon dioxide, and preferably the inert gas is nitrogen.

8. The process according to any one of claims 2 to 7, wherein the treatment of the particulate hydrochlorothiazide with the basic agent in gaseous form consist in:
- flushing the particulate hydrochlorothiazide with the basic agent in gaseous form,
- storing the particulate hydrochlorothiazide in the presence of the basic agent in gaseous form,
- drying the particulate hydrochlorothiazide in the presence of the basic agent in gaseous form,
- mixing the particulate hydrochlorothiazide in the presence of the basic agent in gaseous form, or
- micronizing the particulate hydrochlorothiazide in the presence the basic agent in gaseous form as a milling gas.

9. The process according to claim 5, wherein the treatment of the particulate hydrochlorothiazide with ammonia consists in contacting the particulate hydrochlorothiazide with a solid ammonium salt evolving ammonia during decomposition, preferably with a salt selected from a group consisting of ammonium acetate, ammonium carbonate, ammonium hydrogen carbonate, and mixtures thereof, more preferably with ammonium acetate.

10. The process according to claim 9, wherein the amount of the solid ammonium salt is within a range of 1 - 50% w/w, preferably within a range of 2-5% w/w, in relation to the weight of the particulate hydrochlorothiazide.

11. The process according to claims 9 or 10, wherein the treatment of the particulate hydrochlorothiazide with the ammonium salt, preferably with ammonium acetate, is performed as:
- mixing the particulate hydrochlorothiazide with the solid ammonium salt followed by heating the resulting solid mixture to decompose the ammonium salt while evolving gaseous ammonia, or
- co-micronizing the particulate hydrochlorothiazide with the solid ammonium salt followed by heating the resulting co-micronizate to decompose the ammonium salt while evolving gaseous ammonia.

12. The process according to claim 11, wherein heating of the resulting solid mixture or the resulting co-micronizate is carried out at a temperature withing a range of 50-120°C under vacuum.

13. The process according to claims 2 or 3, wherein the basic agent of step (b) and/or the one or more basic agents of step (c) have the liquid form.

14. The process according to claim 13, wherein the basic agent of step (b) and/or the one or more basic agents of step (c) is selected from an aqueous solution of one or more alkali metal hydroxides, of one or more alkaline earth metal hydroxides, one or more alkali metal carbonates and/or one or more alkali metal hydrogen carbonates, preferably from an aqueous solution of one or more alkali metal hydroxides and/or of one or more alkaline earth metal hydroxides.

15. The process according to claim 14, wherein alkali metal hydroxide is selected from a group consisting of lithium, potassium and sodium hydroxide and alkaline earth metal hydroxide is calcium hydroxide.

16. The process according to claim 15, wherein the basic agent of step (b) and/or the one or more basic agents of step (c) is an aqueous solution of sodium hydroxide.

17. The process according to any of claims 14 to 16, wherein concentration of one or more alkali metal hydroxides and/or one or more alkaline earth metal hydroxides in aqueous solution is within a range of 0,001-10% w/w, preferably within a range of 0,01-5% w/w, more preferably within a range of 0,02-0,5% w/w, in relation to the total weight of the aqueous solution.

18. The process according to any of claims 13 to 17, wherein the treatment of the particulate hydrochlorothiazide with the basic agent in the liquid form consists in:
- washing the particulate hydrochlorothiazide with the basic agent in liquid form during filtration, or
- suspending the particulate hydrochlorothiazide in that basic agent in liquid form followed by filtration.

19. The process according to claim 1, wherein the basic agent of step (b) is an aqueous solution of sodium hydroxide of concentration within a range of 0.001-10% w/w, preferably 0,01-5% w/w, more preferably 0,02-0,5% w/w, in relation to the total weight of the aqueous solution and wherein the treatment of the particulate hydrochlorothiazide consists in washing the particulate hydrochlorothiazide with said aqueous solution of sodium hydroxide during filtration.

20. The process according to claim 1, wherein the basic agent of step (b) is a gaseous mixture of ammonia and nitrogen, wherein a concentration of ammonia in said gaseous mixture is within a range of 0,05 - 10% v/v, preferably 0,1 - 8% v/v, more preferably 0,2 - 3% v/v, in relation to a total volume of said gaseous mixture, and wherein the treatment of the particulate hydrochlorothiazide consists in micronizing the particulate hydrochlorothiazide in the presence of said mixture as a milling gas.

21. A process for reducing amount of 6-chloro-4-nitroso-3,4-dihydro-2H-1,2,4-benzothiadiazide-7-sulfonamide-1,1-dioxide in a particulate hydrochlorothiazide consisting in
- providing the particulate hydrochlorothiazide containing 6-chloro-4-nitroso-3,4-dihydro-2H-1 ,2,4-benzothiadiazide-7-sulfonamide-1,1-dioxide, and
- heating the particulate hydrochlorothiazide to 120-150°C, preferably to 140°C.
